(19) Europäisches Patentamt

European Patent Office

Office européen des brevets

(11) **EP 1 438 944 A2**

(12) **EUROPEAN PATENT APPLICATION**

(43) Date of publication:
**21.07.2004 Bulletin 2004/30**

(51) Int Cl.7: **A61F 13/15**

(21) Application number: **03028445.9**

(22) Date of filing: **11.12.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **12.12.2002 US 317586**

(71) Applicant: **McNEIL-PPC, INC.**
**Skillman, New Jersey 08558 (US)**

(72) Inventors:
- **Erspamer, John**
  **Lakeland, TN 38002 (US)**
- **Hill, Bernard**
  **Memphis, TN 38103 (US)**

- **Lerner, Katja**
  **51107 Cologne (DE)**
- **Poccia, John**
  **Monmouth Beach, NJ 07750 (US)**
- **Loyen, van, Dietmar**
  **67661 Kaiserslauten (DE)**
- **Wessel, Astrid**
  **42105 Wuppertal (DE)**

(74) Representative:
**Groening, Hans Wilhelm, Dipl.-Ing.**
**BOEHMERT & BOEHMERT**
**Pettenkoferstrasse 20-22**
**80336 München (DE)**

(54) **Absorbent article with an odor control coating**

(57) An absorbent article having an odor control coating on the cover or the absorbent core, wherein the coating contains an effective amount of odor control agent. Such odor control coating was found to not only provide superior odor control but also fails to impart discoloration of the substrate to which it is applied.

# FIG. 1A

## Description

### Field of the Invention

The present invention relates to odor control coatings and related articles of manufacture. In particular, the present invention relates to ethylene diamine tetracetate ("EDTA") coatings that control odor without discoloring, e.g., imparting yellow color, the substrate to which it is applied.

### Background of Invention

Odor associated with bodily discharges, such as, menstrual fluid, urine, breast milk, perspiration, and wound discharges, can be effectively eliminated or reduced by many known routes. EDTA is a compound that is used to reduce odors in absorbent articles.

Most odor control materials are available only in powder form, and as such, are dusty and difficult to incorporate and contain in commercial body fluid absorbent products. EDTA is the acid itself and it is only slightly soluble in water. Several salts are available, e.g., Na, $Na_2$, $Na_3$, $Na_4$, that increase solubility of EDTA in water.

One proposed solution to this problem is by means of a polymeric system that facilitates the incorporation of antiodorant powders into sanitary napkins and other absorbent products and contains them in a dust free manner, while allowing good contact with the absorbed body fluid. This polymeric system utilizes hydrophilic, water swellable, water insoluble absorbent polymers (i.e. absorbents and superabsorbents) having dispersed therein the active deodorant powder. However, this solution is time consuming and increases the cost of the absorbent product containing such a system.

Commonly, materials used to make absorbent articles are white or near white. It is known that the use of zeolites are used as odor controlling agents in part because they are white to off-white, so that sheet products having zeolite as the particulate material for absorbing odorous substances can have a clean, unobtrusive appearance. In order to give absorbent articles a white appearance, a colorant, such as titanium dioxide or calcium carbonate, can be added to the material to produce a white color. Usually, sufficient colorant is added to provide opacity sufficient to mask any body discharges, such as, menstrual fluid, urine, breast milk, perspiration, and wound discharges, which are absorbed by the article.

It has been found that coating at least one component of an absorbent article with an aqueous solution of $Na_4$-EDTA produced yellowing of the coated material. Any discoloration of the white or off-white color of the absorbent article, e.g., yellowing, would not be acceptable to users.

What is needed is an odor control agent that is efficient, economical, and does not produce discoloration. It has been surprisingly discovered that an odor control coating that is applied as an aqueous solution of an odor control agent does not produce discoloration and provides not only superior odor control properties, but is also economical to use.

### Summary of the Invention

An absorbent article having an odor control coating that not only controls odor, but also prevents the substrate to which it is applied from discoloration, e.g., yellowing. The coating is applied as an aqueous solution of the odor control agent in aqueous solvent. The coating can be applied to any component of the absorbent article that comes into contact with bodily fluids, excretions and discharges. One useful odor control agent that meets these criteria is $Na_3$-EDTA. Such odor control coating was found to prevent yellowing of the substrate to which it is applied.

### Brief Description of the Drawings

Figure 1A is a top plan view of one embodiment of a disposable sanitary napkin of the present invention.
Figure 1B is a bottom plan view of one embodiment of a disposable sanitary napkin of the present invention.
Figure 2A is an enlarged section taken along line 2-2 of Figure 1A.
Figure 2B is an enlarged section taken of an alternative embodiment along line 2-2 of Figure 1A.
Figure 3 is an enlarged section taken along line 3-3 of Figure 1A.

### Detailed Description

Absorbent articles that are useful in the present invention include any absorbent article that absorbs bodily fluids, excretions and discharges. Such absorbent articles include sanitary napkins, diapers, incontinence devices, breast pads, underarm shields, shoe liners, surgical dressings, adhesive bandages, tampons, and the like.

As used herein, the term "sanitary napkin" is intended to include all products conventionally used to absorb menstrual fluid or vaginal discharge, which are not tampons, whether referred to as sanitary napkins, panty shields, pantiliners or similar or synonymous names.

As used herein, the term "an effective amount of an odor control agent" means an amount of odor control agent that provides an odor control rating of less than about 5.5 on a 10 cm line scale for overall smell intensity for blood and synthetic urine tests at eight hours according to the ISO 8586/1 norm.

The odor control coating of the present invention may be applied to the substrate as an aqueous solution of the odor control material as the solute and a solvent. The solution is then allowed to dry, thereby resulting in a coated substrate.

Useful solvents in the present invention include any solvent in which the solute, e.g., odor control material, is soluble. In particular, it has been found that a polar solvent, such as water, is useful for salts of EDTA.

Useful solutes in the present invention include any solute that has odor control properties and does not impart discoloration, e.g., yellowing of the substrate. Examples of such solutes include chelating or sequestering agents (builders). Chelating agents useful in the present invention include the sodium, potassium and ammonium salts of diphosphoric acid, triphosphoric acid, pyrophosphoric acid, orthophosphoric acid, hexametaphosphoric acid, 1-hydroxyethane-1,1- phosphonic acid, diethylenetriamine penta(methylene diphosphonic acid), ethylenediamine tetraacetic acid (EDTA), diethylenetriamine pentaacetic acid (DTPA), N-(hydroxyethyl) ethylenediamine triacetic acid (HEDTA), propylenediamine tetraacetic acid (PDTA), nitrilotriacetic acid (NTA), mellitic acid, 1,2,3,4-cyclopentane tetracarboxylic acid, succinic acid, lauryl succinic acid, oxydisuccinic acid (ODS), carboxymethyloxysuccinic acid, citric acid, lactic acid, tartaric acid, O-carboxymethyltartronic acid, polyacrylic acid, poly(.alpha.-hydroxyacrylic acid), poly(tetramethylene-1,2-dicarboxylic acid), poly(4-methoxytetramethylene- 1,2-dicarboxylic acid), acrylic acid/maleic acid copolymer (polycarboxylate), acrylic acid/allyl alcohol copolymer (polycarboxylate), sodium PCA, gluconic acid, glucoheptonic acid, lactobionic acid, maltobionic acid, 1-hydroxyethylidene biphosphate, etidronic acid, amino phosphanates, and mixtures thereof. $Na_3$-EDTA is one solute that has been identified as being particularly useful.

Useful substrates in the present invention include any substrate that contacts bodily fluids, excretions and discharges. Such substrates include a cover, a transfer layer, an absorbent core, wings, backsheet layer, and combinations thereof.

Any method of applying the coating solutions to the substrate is useful in the present invention. Such methods include, but are not limited to, dip coating, curtain flow coating method, spin coating, bar coating, roll coating, hand coating, brush coating, spray coating, and the like.

When the present invention is in use, bodily fluids, excretions, and discharges are in intimate contact with the odor control coated substrate, thereby maximizing the efficacy of the odor control properties of the coating. Since the odor control material is coated onto the substrate, it is now possible to design even more effective deodorizing absorbent articles.

More specifically, one useful odor control agent was $Na_3$-EDTA, which is available as Dissolvine® $Na_3$. Without being bound by theory, it is believed that the high pH of $Na_4$-EDTA (pH= about 10) caused oxidation of pulp fibers in a non-woven substrate, which resulted in discoloration, e.g., yellow tint formation. It was found that lowering the pH, whether by using a different salt for of EDTA, e.g., $Na_3$-EDTA (pH= about 8-9), or by adding acid to the high pH EDTA solution did not result in oxidation of pulp fibers in a non-woven substrate as no discoloration was found.

$Na_3$-EDTA is also available as Dissolvine® $Na_3$-36 as a 36% aqueous solution. Other possible options are Dissolvine® $K_3$-123-S ($K_3$-EDTA, 50% solution) and Dissolvine® $AM_3$-40 (($NH_4)_3$-EDTA, 40% slution). Each is available from Akzo Nobel. Versene® $(NH_4)_4$EDTA chelating agent (38% aqueous solution) is available form Dow Chemicals.

It is well within the skill of one in the art to make a $Na_3$-EDTA solution using $Na_4$-EDTA as a starting component. $Na_4$-EDTA is available from many sources including Dow Chemicals (Versene® Powder chelating agent, Versene® Powder A, Versene® 220E, Versene® 220 Crystals Chelating agent, Versene® 100 XL (38 % aqueous solution), Versene® 100 EP (38 % aqueous solution), Versene® 100 (39 % aqueous solution)), Akzo Nobel (Dissolvine® 220-S, Dissolvine® NA-X, Dissolvine® NA, Dissolvine® E-39 (39 % aqueous solution), Dissolvine® 100-S (38 % aqueous solution)), or BASF (Trilon® B and Trilon® BX).

A prediction regarding the amount of EDTA needed can be determined for absorbent products that do not contain any other means of preventing or neutralizing ammonia formed by urea by applying Equation 1:

EQUATION 1

EDTA amount, mg = (Chelation Value mg of EDTA per mg Ca++) * (Concentration

of Ca++ Excreted in Urine) * (Amount of Urine)

Example:

Typical Chelation Value for EDTA = 9.9 g of EDTA needed per 1.0 g of Ca ++

Concentration of Ca++ = 300 mg Ca ++ excreted per 24 hours

Amount of Urine Excreted in 24 hours = 1000 g per 24 hours

Amount of Urine expected on a liner per use = 6.0 grams

EDTA amount, mg = 9.9 g of EDTA * .300 g * 1/1000 grams * 6.0 grams * 1000 mg/l

gram

EDTA amount, mg = 17.8

Because the equation is highly dependent on the assumption that is made for the Ca++ concentration and the amount of urine excreted, the amount of EDTA needed can vary significantly. For example, the amount of EDTA needed can range from a low of 2.97-mg (lowest concentration of Ca++) and a high of 22.3 mg (highest concentration of Ca++). The above equation is meant to be used as a general understanding to determine appropriate starting levels of EDTA for an absorbent article.

Some absorbent articles can contain components that can neutralize ammonia formed from urea. A typical component found in some absorbent articles is a cross-linked polymer of partially neutralized acrylic acid. Because the polymer is partially neutralized the free acid groups can be used to neutralize ammonia. Although there is not enough buffering capability to completely neutralize all the ammonia that can be produced by urea, a combination of superabsorbent polymers and EDTA can be used together to help eliminate the ammonia odor. Because of the buffering capability of the superabsorbent polymers, a lower amount of EDTA can be used in these systems. To determine the new amount of EDTA, the mole fraction of the potential amount of ammonia remaining after neutralization of the superabsorbent polymer can be used to determine the minimum amount of EDTA needed.

EQUATION 2

f (mole fraction of ammonia remaining) = ((Total potential ammonia from Urea) -

(Ammonia neutralized by acrylic acid)/Total potential ammonia formed from Urea))

EQUATION 3

(for absorbent articles containing a partially neutralized polyacrylate polymer)

EDTA amount needed, mg = Equation 2 * Equation 1

<u>Example</u>

Typical Chelation Value for EDTA = 9.9 g of EDTA needed per 1.0 g of Ca ++

Concentration of Ca++ = 300 mg Ca ++ excreted per 24 hours

Amount of Urine Excreted in 24 hours = 1000 g per 24 hours

Amount of Urine expected on a liner per use = 6.0 grams

Amount of Urea excreted in 24 hours = 25 grams

% Free Acid groups in a partially neutralized polyacrylate superabsorbent = 25%

Amount of SAP in a liner = .2 grams

EQUATION 2

f (mole fraction of ammonia remaining) = ((Total potential ammonia from Urea)-

(Ammonia neutralized by acrylic acid)/Total potential ammonia formed from Urea))

f = (Urine amount, g x (Urea, g/g of Urine) x 1/MW Urea * 2 mol NH3/l mol of Urea))

- ((Superabsorbent amount, g/ MW of Acrylic Acid)* % acid groups/100))

f = (6g * (25g/1000g)*(1/60 g/mol) * 2 mol NH3) - ((.2 g/72 g/mol) * (25/100))

f=.86

EQUATION 3

(for absorbent articles containing a partially neutralized polyacrylate polymer)

EDTA amount needed, mg = Equation 2 * Equation 1

Or

EDTA amount needed, mg = f (Equation 1)

Or

EDTA amount needed, mg = .86 (17.8 mg)

EDTA amount needed, mg = 15.3

Equation 3 shows that for absorbent articles containing a partially neutralized salt of acrylic acid, a lower amount of EDTA is needed, e.g., 15.3 mg versus 17.8, for articles that do not contain the superabsorbent. The calculation can be used to optimize cost and performance when designing absorbent articles that contain both EDTA and superabsorbent polymers.

The range of EDTA needed in absorbent articles can be from about 2.97 mg to about 22.3 mg based on the varied amounts of urine excreted and calcium ions present. To determine a general concentration for absorbent articles the low range and high range of EDTA needed would be divided by the absorbent capacity of the absorbent article as determined by any number of methods known in the art, e.g., in vitro and in vivo.

As a non-limiting example, a pantyliner has an absorbent capacity value of 10 grams. The EDTA concentration would be 2.97 mg EDTA/10 grams or .297 mg of EDTA/gram of capacity (low) to 22.3/10 grams or 2.23 mg/gram of capacity of the absorbent article (high).

While the above equation exemplified EDTA, the amount of any other odor control agent could be similarly calculated based upon, e.g., the amount of calcium to be chelated. In addition, the amount of any other salt of EDTA or other control agent can also be calculated.

Absorbent articles that are useful in the present invention are often composed of layers of materials, e.g. many sanitary napkins and disposable diapers have a body-facing layer, a backing layer and an inside absorbent layer. The odor controlling coating of the present invention can easily be incorporated in any desired location in the body fluid absorbent structure. For example, where a suitable non-woven web or impervious film already is being used as part of the total sanitary napkin structure, it first can be coated with a solution containing an appropriate odor controlling solute and then used as before in the sanitary napkin construction.

The actual placement of the coated substrate in the absorbent product structure is not critical to the instant invention, and it can be placed wherever it is wished to have the odor control solute available to contact the body fluid as it is absorbed in actual use.

Many commercially available types of sanitary napkins are constructed somewhat similarly to that depicted in U.S. Patent No. 4,217,901, which has a (1) an absorbent layer (made of any suitable absorbent material, e.g., comminuted wood pulp fibers, cotton linters, rayon fiber, cotton staple etc.) as the absorbent core, (2) a body fluid impervious backsheet (made of, e.g., polyethylene or polypropylene film) on the undergarment facing side of the absorbent pad, which is provided to preclude absorbed body fluid from striking through and wetting or staining the undergarment. (3) A sheet of tissue paper surrounding the absorbent pad, (4) a body fluid pervious cover (made of any of the commonly used absorbent product covers such as gauze, non- woven material reinforced with adhesive binders etc.) enveloping the tissue paper wrapped absorbent pad and body fluid impervious cover, and (5) an optional adhesive strip on the undergarment facing surface for those products designed to be adhesively attached to the wearers undergarment. Additionally, absorbent articles often include a transfer layer, which is positioned between the cover and absorbent core. The transfer layer may be any material that draws or transports fluid away from the cover and body towards the absorbent core or layer.

While sanitary napkins often contain the above construction, such construction is not critical to the present invention. While the above construction delineates various layers, separate layers can be integrated into a unitized construction. For example, the cover and absorbent layer can have a unitized construction wherein only one layer would function as both the cover and the absorbent layer.

The odor control coating-containing substrate of the present invention can be used as a layer directly under the cover of a sanitary napkin. There are many other constructions known for sanitary napkins, panty liners, etc., some omitting the tissue paper (3) above, but most constructions do have an impervious backsheet(2) and an absorbent core (1). The cover and the backsheet encase all components of the article. The cover and the backsheet are typically joined or sealed to each other along their peripheral edge using any method known in the art.

The substrates of the present invention can be used in various places in the sanitary napkin, the choice of which would dictate the specific substrate desired, and they can even replace some of the required layers. Thus the water-insensitive film substrate could be used as the impervious layer (2) above, while the tissue paper substrate could replace that of (3) above, or could be used as a separate layer underneath the tissue paper (3), while the non-woven fiber substrate could be underneath the body fluid pervious cover of, (4) above, or be placed on either side of, or even in the inside of, the absorbent pad (1) above.

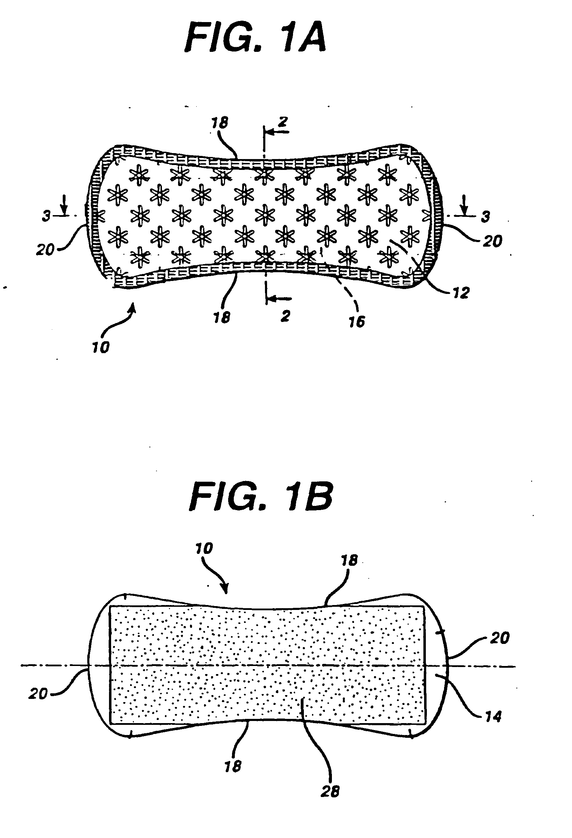

Turing to FIGS. 1A and 1B, sanitary napkin 10 has a porous, body-side cover 12, a liquid-impervious barrier layer 14, and an absorbent layer 16 therebetween. The article has lateral sides 18, and longitudinal ends 20.

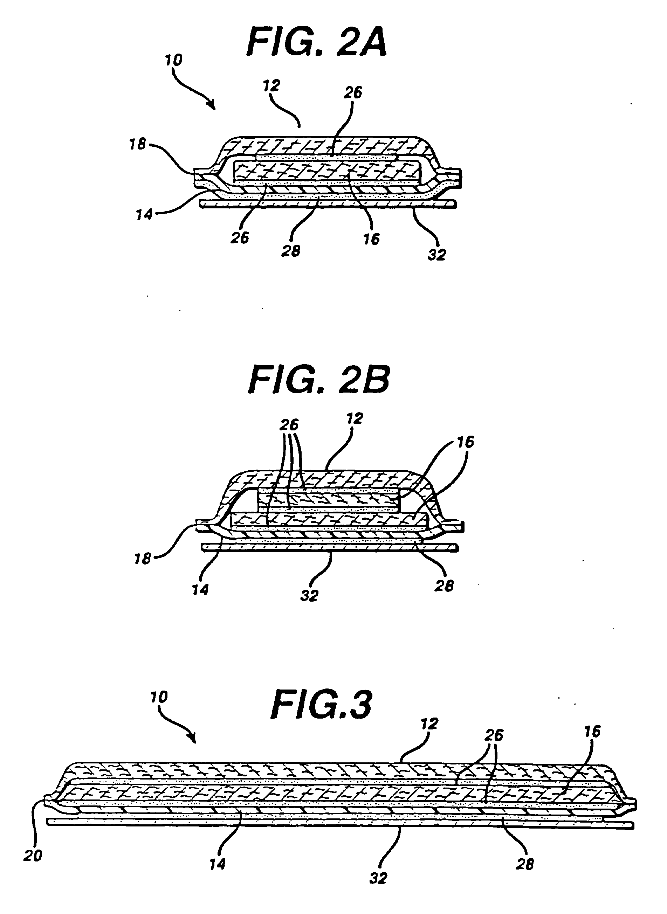

FIGS. 2A and 2b are alternate view along line 2-2 of Fig. 1A depicting two different embodiments as a cross-sectional view of the components making up sanitary napkin 10. In particular, cover 12 is attached to backsheet 14 at lateral side 18. Positioning adhesive 28 is deposited on backsheet 14. Release strip 32 protects positioning adhesive 28. Absorbent core 16 lies between cover 12 and backsheet 14. In FIG. 2B there is depicted two absorbent cores/layers 16. Construction adhesive 26 is depicted as being used to secure cover 12 and backsheet 14 to absorbent core 16 and, in FIG. 2B, both absorbent cores 16 to each other.

Fig. 3 is an alternate view along line 3-3 of Fig. 1A depicting the cross-sectional view of the components making up sanitary napkin 10. In particular, cover 12 is attached to backsheet 14 at longitudinal end 20. Positioning adhesive 28 is deposited on backsheet 14. Release strip 32 protects positioning adhesive 28. Absorbent core 16 lies between cover 12 and backsheet 14. Construction adhesive 26 is depicted as being used to secure cover 12 and backsheet 14 to absorbent core 16.

Cover

The cover 12 typically overlays the absorbent core 16. The exterior of the cover forms the body-facing surface of the sanitary napkin 10. As known by those skilled in the art, the cover may be formed from any fluid pervious material that is comfortable against the skin and permits fluid to penetrate to the absorbent core 16, which retains the fluid. The cover 12 should retain little or no fluid to provide a relatively dry surface next to the skin when in use. A variety of cover materials are known in the art, and any of these may be used. For instance, the cover has been made from fibrous non-woven fabrics made of fibers or filaments of polymers, such as polyethylene, polypropylene, polyester, or cellulose, and combinations or mixtures thereof. The fiber or filament can be single denier or multidenier.

Other materials used in making covers include gauze or for example, a nonwoven material such as the ones described in U.S. Patent No. 3,554,788 (Fechillas), any known porous material with a suitable body contacting surface, including, but not limited to nonwoven webs, apertured films, plastic nets, and the like. The cover 12 could also be made from a fibrous nonwoven composite of bicomponent fibers and pulp fluff.

Alternatively, the cover 12 may be formed from an apertured polymeric film. In addition, such a film may be treated with a surfactant to increase hydrophillicity.

Generally, the cover 12 is a single sheet or layer of material having a width sufficient to form the body-facing surface of the absorbent article. The cover 12 may be longer and wider than the absorbent core.

The cover 12 may be embossed with shapes within a given area. For example, a series or a number of features, e.g., circles, triangles, squares, lines, honeycomb, diamond, floral, etc. are embossed over the entire length and width of the outer surface of web. Each embossed feature has a major and minor axis extending therethrough, the major axis length being greater or equal to the minor axis length. The embossed features may be in a repetitive pattern.

Bicomponent fibers are known in the art and are composed of two polymers with different melting points. At least a portion of the outer surface of each bicomponent fiber has the lower melting polymer. The two polymers may be arranged such that a cross-section of the fiber shows the two polymers in a side-by-side array. Alternatively, the polymers may be positioned in a so-called sheath/core arrangement, in which a core of higher melting polymer is surrounded by a sheath of lower melting polymer. A useful bicomponent fiber is BASF "BICO" code 1050 fiber, a 3.0 denier, 1.5" long staple fiber made of a polyester core and a high density polyethylene sheath (BASF Corporation, Charlotte, North Carolina). Similar fibers (polyethylene sheath and polypropylene core) are available as Danaklon ES-C or ES Bico (Danaklon A/S, Varde Denmark). Pulp fibers may be obtained as IP "'SUPERSOFT" ELM. supplied by the International Paper Company (Memphis, Tennessee), "'RAYFLOC" XJ-HM E-Type Cellulosic Fluff Pulp, (ITT Rayonier), or Korsnas Vigorfluf-EN White (KorsncAs, Gavle, Finland).

Absorbent Core

The absorbent core or layer 16 of the present invention may contain any known absorbent materials including, but not limited to, absorbent fibers, such as cellulose fibers, including, but not limited to wood pulp, regenerated cellulose fibers, and cotton fibers, rayon fibers and the like; superabsorbent fibers or particles; other naturally occurring absorbent materials, such as peat moss; and other synthetic absorbent materials, such as foams and the like. The absorbent layer may also include one or more of the following: thermoplastic binder fibers, latex binder, perfumes, oils or odor-controlling compounds. Additionally, the absorbent layer may be a mixture of two or more types of thermoplastic fibers having different melting points. Bicomponent fibers, fibers with an inner core of a thermoplastic fiber, e.g., polyester, surrounded by an outer sheath of thermoplastic, e.g., polyethylene, having a melting point much lower than the core, have been found to be the best fibers to work with from processing and performance standpoints. Upon application of heat and pressure sufficient to melt at least one of the fiber types, the remaining unmelted fibers will be thermobonded or fused together into a porous web.

Cellulosic pulp fibers can also be included with thermoplastic fibers. Since thermoplastic fibers, without further treatment, are essentially hydrophobic, the absorbent layer will not effectively draw fluid away from the composite cover and transfer layer absent some hydrophilic material. It is important to have sufficient pulp to absorb fluid.

An absorbent structure may be delivered in roll-goods form, or in other packaging formats such as festooning, and is particularly useful as an absorbent core for disposable absorbent articles such as diapers, adult incontinence pads and briefs, and feminine sanitary napkins.

In one useful embodiment, the structure of the present invention is prepared as an airlaid web. The airlaid web is typically prepared by disintegrating or defiberizing a cellulose pulp sheet or sheets, typically by hammermill, to provide individualized fibers. The individualized fibers are optionally mixed with functional particles, and are then air conveyed to one or more forming heads on the airlaid web forming machine. The forming head then deposes a stratum in the forming wire. A stratum may contain, for example, cellulose fibers, SAP and other functional particles, and bicomponent fibers.

In some embodiments, the structures of the invention contain a carrier tissue. The use of a compaction roll prior to the introduction of the particle areas eliminates the need for the tissue.

Unit operations involve the use of multiple forming heads, for example, up to four, five, six or seven forming heads may be used to provide additional strata to the web. Several manufacturers make airlaid web forming machines, including M&J Fibretech of Denmark and Dan-Web, also of Denmark. The forming heads include rotating drums, or agitators generally in a racetrack configuration, which serve to maintain fiber separation until the fibers are pulled by vacuum onto a foraminous condensing drum or foraminous forming conveyor, or forming wire. For example, in machines manufactured by M&J Fibretech, the forming head includes a rotary agitator above a screen. Other fibers, such as a synthetic thermoplastic fiber, may also be introduced to the forming head through a fiber dosing system, which includes a fiber opener, a dosing unit and an air conveyor.

The airlaid web is transferred from the forming wire to a calender or other densification stage to densify the web, increase its strength and control web thickness. The fibers of the web may alternatively, or additionally, be bonded by application of a binder or foam addition system, followed by drying or curing. As a result, heat seals between the thermoplastic material and the fibers of the various strata are formed. The finished web is then rolled for future use.

As contemplated by the present invention, one or more forming heads of the airlaid web forming machine distributes the desired fiber for the various strata of the absorbent core or structure. For example, a first forming head may be used to provide a first fibrous stratum, for example a stratum comprising a cellulose fiber, bicomponent fiber, and optionally a carrier tissue. The first stratum may be a wicking stratum.

Functional particles may optionally (or additionally) be applied to the strata by particle applicator. Thus, SAP particles or other functional particles are thus applied. Optionally the strata can be compacted or densified in a nip formed by a pair of calender rolls. The fibers may be compressed to the desired thickness and density. The lower stratum may be compacted at this point in the manufacturing process to close the pores of the web if the particles are fine, and to prevent spillage on to the forming wire.

Additional strata can then be formed on top of lower strata in the same manner the first stratum is formed, by use of forming heads, optionally particle applicators, and optionally nips formed by calender rolls.

The airlaid web is transferred from the forming wire and is compacted or densified, for example, by use of a calender or to increase its strength and control web thickness. Preferred ranges of densification are from about 0.035 to about 0.50 g/cc, more preferably about 0.050 to about 0.50 g/cc, even more preferably about 0.20 g/cc. The web is then subjected to further treatment including pressure, heat and/or the application of a binder. For example, a binder (such as a spray or foam binder), may be applied at binder applicators, which may be disposed after the calendar. A series of ovens also may be used in processes of the invention, after application of the binder, for drying, curing or thermal bonding. The airlaid structure may be heated to a temperature in the range of from about 125 to about 180°C. A further overall binder may then be applied to the structure. This binder can be applied by spray, foam or mist, and is applied to reduce dust-off on the surface of the structure.

The air laid structure may be heated in additional ovens at a temperature in the range of from about 125 to about 180°C. The airlaid structure may be treated at pressure in the range of from about 0.1 to about 10 psi, preferably about 1.5 psi.

The finished web is then rolled for future use. This continuous band of fibrous web can be slit or cut to form individual absorbent articles in a cutting unit, which has not been depicted in this figure.

Optionally, the finished web may be slit or perforated at the heat seal to yield narrow slit core material having a heat seal along both edges. The heat seals to be slit must be of sufficient width to provide two effective seals after slitting.

In other embodiments, various other strata containing other types and amounts of fibers may be applied above or below the upper and lower strata of the structure of the present invention. For example, the absorbent article may contain also a fluid previous top sheet and a fluid impervious backsheet. Exemplary absorbent articles that can be formed from absorbent cores of the invention include diapers, sanitary napkins, and adult incontinence products.

In a useful embodiment, the absorbent layer is made from airlaid pulp and includes about 15% by weight of superabsorbent polymer available as Hysorb CL 15 from BASF. The amount of superabsorbent in the absorbent core may be in the range of from about 5 to about 50 % by weight of the absorbent core. The absorbent core may be compressed or uncompressed, embossed, or calendered.

The absorbent core 16 may also be coated with the odor control coating of the present invention.

The absorbent layer 16 may have one or more layers in an overlapping or side-by-side arrangement. See for example, Fig. 2B which is an alternative view along line 2-2 of Fig. 1A. These multiple absorbent layer layers may be of similar width and thickness or varying dimensions.

As indicated above, the absorbent core 16 is preferably located about the central longitudinal axis of the absorbent article. The absorbent core 16 may be formed of a material having parallel sides, or the absorbent core material may have a varying width to correspond to an absorbent pad having a varying width along the length of the sanitary napkin 10.

Backsheet

Backsheet 14 may be located adjacent to the absorbent core 16 and to the cover layer 12 in portions elsewhere. The backsheet 14 of the present invention is a body fluid impervious material, typically referred to as a "barrier," which is at least substantially impermeable to liquids. Its exterior forms the garment-facing surface of the absorbent article. The backsheet may be any thin, flexible, body fluid impermeable material, such as, but not limited to, a polymeric film, e.g., polyethylene, polypropylene, or cellophane, or a normally fluid pervious material that has been treated to be impervious, such as impregnated fluid repellent paper or non-woven material, including non-woven fabric material, or a flexible foam, such as polyurethane or cross-linked polyethylene.

Optionally, the backsheet 14 may be breathable, i.e., permits vapor to transpire. Known materials for this purpose include nonwoven materials, monolithic and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

Bonding Methods

The layers of the absorbent article may be, but not necessarily, bonded, e.g., glued or adhered, to the adjacent layer. For example, the underside of the cover 12 composite may be adhered to the top side of the absorbent layer 16. The underside of the absorbent layer 16 is adhered to the top side of the backsheet 14. As previously stated, any methods known in the art, such as fusion bonding, adhesive attachment, or by any other securement means can be used to secure the individual layers together to form the final absorbent article. Included within such methods are co-embossing, thermobonding, mechanical bonding, and the like. Fusion bonding includes heat bonding, ultrasonic bonding, and the like.

Adhesive is typically used to attach the layers into a single absorbent article. For example, in one embodiment, the body facing cover 16 is attached to the barrier with adhesive HL 1491 available from H.B Fuller and Company (St. Paul, MN). The adhesive may be applied in any method.

Adhesive may include pressure sensitive adhesive that is applied as strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include KratonTM elastomers from Shell Chemical Company, VectorTM elastomers from Dexco, SolpreneTM from Enichem Elastomers and StereonTM from Firestone Tire & Rubber Co.; hot melt adhesive based on olefin polymers and copolymers where in the olefin polymer is a terpolymer of ethylene and a co-monomers, such as vinyl acetate, acrylic acid, methacrylic acid, ethyl acrylate, methyl acrylate, n-butyl acrylate vinyl silane or maleic anhydride. Commercial examples of these types of polymers include Ateva( polymers from AT plastics), Nucrel( polymers from DuPont), Escor (from Exxon Chemical).

In an embodiment of the present invention, the layers 12, 14, and 16 of the sanitary napkin 10 may be attached to one another to form a cohesive unit to enhance the napkin's stability. In its preferred embodiment, the sanitary napkin 10 has construction adhesive 26 present between the cover layer 12 and the absorbent layer 16 and also present between the absorbent layer 16 and the backsheet 14. The construction adhesive 26 serves to hold the layers 12, 14, 16 together and to minimize pad deformation during use. The construction adhesive 26 may be a hot melt adhesive supplied by the H.B. Fuller Company (St. Paul, Minnesota) as code HL 1308x. The adhesive can be applied as either a thin porous film, or in a random spray, in a controlled spiral pattern, or in any other application pattern. The adhesive coat weight is approximately 10 mg./in $^2$.

Secure attachment of absorbent article of the claimed invention to the garment contributes to maintaining the feeling of the user that the absorbent article and the garment are one in the same, i.e., permits the absorbent article to move with the underwear.

The absorbent article of the present invention may be applied to the crotch by placing the garment-facing surface against the inside surface of the crotch of the garment. Various methods of attaching absorbent articles may be used. For example, chemical means, e.g., adhesive, and mechanical attachment means, e.g., clips, laces, ties, and interlocking devices, e.g., snaps, buttons, VELCRO (Velcro USA, Inc., Manchester, NH), zipper, and the like are examples of the various options available to the artisan.

Additionally, positioning adhesive 28 may be applied to the garment facing side of the absorbent article. The positioning adhesive 28 may be any adhesive known in the art. As a non-limiting example, pressure sensitive adhesive strips, swirls, or waves may be applied to help maintain the absorbent article in place. As used herein, the term pressure-sensitive adhesive refers to any releasable adhesive, or releasable tenacious means. Suitable adhesive compositions, include, for example, water-based pressure-sensitive adhesives, such as acrylate adhesives. Alternatively, the adhesive composition may include rapid setting thermoplastic "hot melt," rubber adhesives, two-sided adhesive tape, and the like.

Where positioning adhesive 28 is used on the garment facing side of the backsheet 14, as depicted in FIGS 2A, 2B, and 3, a release strip 32 may be applied to protect the adhesive on the absorbent article prior to attaching the absorbent article to the crotch. The release strip 32 can be formed from any suitable sheet-like material that adheres with sufficient tenacity to the adhesive to remain in place prior to use but which can be readily removed when the absorbent article is to be used. Optionally, a coating may be applied to release strip 32 to improve the ease of removability of the release strip 32 from the adhesive. Any coating capable of achieving this result may be used, e.g., silicone.

Transfer Layer

Optionally, the absorbent article of the present invention may include a transfer or distribution layer. If included, the transfer layer may be made of any known material that will take up fluid and then distribute and release it to an adjacent absorbent layer for storage. Transfer layers have a relatively open structure that allows for movement of fluid within the layer. Suitable materials for such transfer layers include fibrous webs, resilient foams, and the like.

The mass of materials making up the transfer layer may be absorbent, although the materials themselves are not absorbent. Thus, transfer layers that are made of hydrophobic, nonabsorbent fibers may be able to accept large volumes of fluid into interfiber void spaces while the fibers themselves do not absorb any significant quantities of fluid. Likewise, open-celled foam structures that are made from nonabsorbent materials may also absorb fluid into the cells of the foam. The walls of the cells, however, do not absorb any fluid. The cumulative spaces within the transfer layer, i.e., the interfiber void spaces in the fibrous transfer layer or the open cells in the foam transfer layer, function much like a container to hold fluid.

Typically, transfer layer fibrous webs are made of resilient, nonabsorbent materials to provide void volume and to allow for free movement of fluid through the structure. Transfer layers that are made from webs of mostly absorbent fibers absorb the fluid as it enters the structure and do not distribute it throughout the rest of the structure as efficiently as webs containing non-absorbent materials.

Transfer layers that are made from webs of mostly absorbent fibers absorb the fluid as it enters the structure and do not distribute it throughout the rest of the structure as efficiently as webs containing non-absorbent materials. Preferred transfer layer fibrous webs include nonabsorbent materials to provide void volume and to allow for free movement of fluid through the structure. Examples of preferred materials include polypropylene, polyethylene, polyester, bicomponent materials, nylon and mixtures or combinations thereof. In a preferred embodiment, the transfer layer is an apertured film made from a carbon black pigmented polyethylene. An example useful in the present invention is a black topsheet film available from the ADMA division of Tredegar (Chieti, Italy) as 33-12-2001.

The transfer layer does not have to be apertured film; it can be any other pigmented nonwoven material, such as, foam or netting, which transports fluid and in combination with the cover, provides masking of the absorbent core. However, in one embodiment, the transfer layer is a 25 gsm apertured film made from polyethylene.

In one embodiment, the cover and transfer layers are joined together by first applying adhesive to the underside of the cover material and placing the cover web onto the transfer layer material. While any adhesive may be used, such as any non-pressure sensitive adhesive, a preferred adhesive is D 1280 BE (available from Fuller Co., Germany)

The composite layer formed from the cover and transfer layers may be further processed. In a preferred embodiment, the composite layer includes an embossed pattern on the outer cover surface. For example, flowers and rails under the CAREFREE® pattern (e.g., U.S. Design Patent No. 439,057) are embossed after the composite is formed, which results in an embossed pattern having flowers, rails, and squares

Wings

Wings, also called, among other things, flaps or tabs, may also be part of the absorbent article of the present invention. Wings and their use in sanitary protection articles is described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. The disclosures of these patents are incorporated herein by reference in their entirety. As disclosed in the above documents, wings are generally speaking flexible and configured to be folded over the edges of the underwear so that the wings are disposed between the edges of the underwear.

**Miscellaneous**

Any or all of the cover, absorbent layer, transfer layer, backsheet layer, and adhesive layers may be colored. Such coloring includes, but is not limited to, white, black, red, yellow, blue, orange, green, violet, and mixtures thereof. Color may be imparted according the present invention through dying, pigmentation, and printing. Colorants used according the present invention include dyes and inorganic and organic pigments. The dyes include, but are not limited to, anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like.

Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

The absorbent article may include other known materials, layers, and additives, such as, foam, net-like material, perfumes, medicaments or pharmaceutical agents, moisturizers, odor control agents, and the like. The absorbent article can optionally be embossed with decorative designs.

The absorbent article may be packaged as unwrapped absorbent articles within a carton, box or bag. The consumer withdraws the ready-to-use article as needed. The absorbent article may also be individually packaged (each absorbent article encased within an overwrap).

Also contemplated herein include asymmetrical and symmetrical absorbent articles having parallel longitudinal edges, dog bone- or peanut-shaped, and the like.

The absorbent article of the present invention may be used with conventional underwear or may be shaped to conform to thong garments. As used herein, the term thong garment includes, but is not limited to, thong underwear, thong swimming suit bottom, G-strings, Rio cut underwear, Rio-cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself.

From the foregoing description, one skilled in the art can ascertain the essential characteristics of this invention, and without departing from the spirit and scope thereof, can make various changes and modifications. Embodiments set forth by way of illustration are not intended as limitations on the variations possible in practicing the present invention.

**EXAMPLES**

Example 1:

Control: Absorbent material was produced at 175 gsm in total basis weight and a density of 0.09 g/cc. The absorbent material was produced using the Dan Web forming head air laid technology. The material was made of 55% Rauma manufactured cellulosic fiber, 27% Trevira® bicomponent fibers, 15% Salsorb-CL15 superabsorbent powder from BASF, and 3% AF-192 latex adhesive from Air Products.

Sample K: Versene® 100XL from Dow Chemical is available as a 38% aqueous solution of $Na_4$-EDTA. This aqueous solution was diluted with water to 20% prior to spraying. The absorbent material was produced using the Dan Web® forming head air laid technology. The absorbent substrate had a total basis weight of 195 gsm and a density of 0.10 g/cc. It was composed of 49% Rauma manufactured cellulosic fiber, 24% Trevira® bicomponent fibers, 13% Salsorb-CL15 superabsorbent powder from BASF, and 3% AF-192 latex adhesive from Air Products. The sodium bicarbonate, BICAR® CODEX 27/50 from Solvay, was applied using the Walkisoft powder dispensing system in the middle layers of the substrate. The Versene 100XL solution was applied on one side of the substrate using the Walkisoft spray system during the manufacturing of the absorbent article. The material was produced with 5.0 gsm EDTA (dry) and 15 gsm of sodium bicarbonate powder.

Sample E: Versene® 100XL from Dow Chemical is available as a 38% aqueous solution of $Na_4$-EDTA. This aqueous solution was diluted with water to 20% prior to spraying. The absorbent material was produced using the Dan Web® forming head air laid technology. The absorbent material had a total basis weight of 180 gsm and a density of 0.10 g/cc. It was made of 54% Rauma manufactured cellulosic fiber, 26% Trevira® bicomponent fibers, 15% Salsorb-CL15 superabsorbent powder from BASF, and 3% AF-192 latex adhesive from Air Products. The Versene ®100XL solution was applied on one side of the substrate using the Walkisoft spray system. The material was produced with 5.0 gsm EDTA (dry).

Sample D: Versene® 100XL from Dow Chemical is available as a 38% aqueous solution of $Na_4$-EDTA. This aqueous solution was diluted with water to 20% prior to spraying. The absorbent material was produced using the Dan Web® forming head air laid technology. The absorbent substrate had a total basis weight of 178 gsm and a density of 0.10 g/cc. It was made of 54% Rauma manufactured cellulosic fiber, 27% Trevira® bicomponent fibers, 15% Salsorb-CL15 superabsorbent powder from BASF, and 3% AF-192 latex adhesive from Air Products. The Versene 100XL solution was applied on one side of the substrate using the Walkisoft spray system. The material was produced with 2.5 gsm EDTA (dry).

Sample I: Sodium bicarbonate, BICAR® CODEX 27/50 from Solvay, and the Versene 220 Chrystal Chelating agent, $Na_4$ salt of EDTA were pre-mixed in a ratio of 25% EDTA and 75% sodium bicarbonate. The absorbent material was produced using the Dan Web® forming head air laid technology. The absorbent substrate had a total basis weight of 196 gsm and density of 0.11 g/cc. It was made of 49% Rauma manufactured cellulosic fiber, 24% Trevira® bicomponent fibers, 13% Salsorb-CL15 superabsorbent powder from BASF, and 3% AF-192 latex adhesive from Air Products. The superabsorbent powder from BASF was applied separately from the EDTA-bicarbonate blend. The powder blend was applied at 10% in the middle layers using the Walkisoft dispensing system during the process of the material. The material was produced with 5 gsm EDTA (dry), 15 gsm $NaHCO_3$.

Sample C: The Salsorb-CL15 superabsorbent powder and the Versene 220 Chrystal Chelating agent, $Na_4$ salt of EDTA were pre-mixed in a ratio of 16% EDTA and 84% superabsorbent powder. The absorbent material was produced using the Dan Web® forming head air laid technology. The absorbent substrate had a total basis weight of 180 gsm and a density of 0.11g/cc. It was composed of 18% Rauma manufactured cellulosic fiber, 8.8% Trevira® bicomponent fibers, and 3% AF-192 latex adhesive from Air Products. The EDTA-SAP powder blend was applied at 17% in the middle layers using the Walkisoft dispensing system during the process of the material at 17% of the total weight.

Option 1: Dissolvine® Na3-36 is available as a 36% aqueous solution of $Na_3$-EDTA. This aqueous solution was diluted with water to 15% prior to spraying. The absorbent material was produced using the Dan Web® forming head air laid technology. The absorbent substrate had a total basis weight of 175 gsm and a density of 0.10 g/cc. It was composed of 55% Rauma manufactured cellulosic fiber, 27% Trevira® bicomponent fibers, 15% Salsorb-CL15 superabsorbent powder from BASF, and 3% AF-192 latex adhesive from Air Products. The Dissolvine® Na3-36 solution was applied on one side of the substrate using the Walkisoft spray system. The material was produced with 2.5 gsm EDTA (dry).

Option 2a: The Dissolvine® Na3-36 is available as a 36% aqueous solution of $Na_3$-EDTA. This aqueous solution was diluted with water to 15% prior to spraying. The absorbent material was produced using the Dan Web® forming head air laid technology. The absorbent substrate had a total basis weight of 180 gsm and a density of 0.10 g/cc. It was composed of 51% Rauma manufactured cellulosic fiber, 25% Trevira® bicomponent fibers, 14% Salsorb-CL15 superabsorbent powder from BASF, and 3% AF-192 latex adhesive from Air Products. The sodium bicarbonate, BICAR® CODEX 27/50 from Solvay, was applied at 6%using the Walkisoft powder dispensing system in the middle layers of the substrate. The Versene 100XL solution was applied on one side of the substrate using the Walkisoft spray system during the manufacturing of the absorbent article. The material was produced with 2.5 gsm EDTA (dry) and 12 gsm of sodium bicarbonate powder.

**Example 2**

The color of the coated absorbent core inserts made in Example 1 were determined using a Minolta Chroma-Meter CR 300 according to the Manufacturer's Guide as follows:

    1. Switch Apparatus ON

2. INDEX SET: Print = Y

Color Space = N
Scroll-Taste ↺↻
Data Protect = Y
Multi Measure = Y
Scroll-Taste ↺↻
Multi Cal. = N
Scroll-Taste ↺↻
Light Source = D65
ENTER

3. CALIBRATE

Add Standard values for D65 Light source into display
MEASURE white Standard area in the small black box (3 measurements)

4. Choose Color system : COLOR SPACE SELECT

mark ⬅ ➡ L*a*b, set ABS-operating

5. Procedure - each absorbent core insert was measured at five different places along its length for both the top side and the bottom side. These 5 values were averaged. Two different control inserts were used as a comparison, which had no EDTA applied.

The following results were obtained:

Table 1

| SAMPLE | Measurement Orientation | White Average L | Green Average a | Yellow Average b |
|---|---|---|---|---|
| A CONTROL | Top side | 89.466 | -0.172 | 1.02 |
| | Bottom side | 89.694 | -0.17 | 1.002 |
| K 5gsm Na$_4$EDTA spray, 15gsm N$_2$HCO$_3$, dosed separately | Top side | 90.834 | -0.274 | 5.416 |
| | Bottom side | 91.408 | -0.138 | 2.41 |
| E 5gsm Na$_4$EDTA spray | Top side | 88.566 | -0.276 | 3.298 |
| | Bottom side | 88.764 | -0.124 | 1.2 |
| D 2.5gsm Na$_4$EDTA spray | Top side | 88.624 | -0.248 | 2.892 |
| | Bottom side | 89.228 | -0.148 | 1.254 |
| I 5gsm Na$_4$EDTA, 15gsm NaHCO$_3$, Bicarb/ EDTA blend | Top side | 90.176 | -0.114 | 1.126 |
| | Bottom side | 89.904 | -0.128 | 1.136 |
| C 5gsm Na$_2$EDTA, SAP/EDTA blend | Top side | 88.742 | -0.152 | 1.09 |
| | Bottom side | 88.812 | -0.148 | 1.038 |
| 2$^{nd}$ Control | Top side | 91.518 | -0.098 | 1.282 |
| | Bottom side | 91.414 | -0.126 | 1.39 |
| Option 1 Na$_3$ EDTA 2.5gsm spray | Top side | 91.214 | -0.136 | 1.724 |
| | Bottom side | 91.534 | -0.146 | 1.384 |

Table 1   (continued)

| SAMPLE | Measurement Orientation | White Average L | Green Average a | Yellow Average b |
|---|---|---|---|---|
| Option 2a Na$_3$EDTA 2.5gsm spray + baking Soda 12gsm | Top side | 91.802 | -0.124 | 2.038 |
| | Bottom side | 91.918 | -0.184 | 1.592 |

The results for the L (white = 100 / black = 0) and for the red-green tendency a (-a = green / +a = red) are not significantly different for any of the inserts tested. The blue-yellow tendency b (-b = blue / +b = yellow) however do vary.

As can be seen by the above results, the absorbent core inserts having the Na$_4$EDTA coating became the most yellow on the top side, which was where the aqueous odor control solution was applied by spraying. The absorbent core inserts having solid Na$_4$EDTA did not yellow significantly. The absorbent core inserts having a Na$_3$EDTA coating did not yellow significantly.

**Example 3**

The odor control efficacy was proven in a sensory sniff test involving a panel of 15 trained expert sniffers. The odor profile of pantiliner products was evaluated after inoculating the surface of the pads with bacteria and lamb's blood or synthetic urine, respectively. The differences of the overall urine odor and blood odor and of ammonia odour (urine) and degraded blood (blood) were defined quantitatively 1h and 8h after inoculation. Multiple comparison tests (DUNCAN tests) were used to identify the statistical differences between samples.

Samples: Control, Option 1 and Option 2a where made according to Example 1 above. Comparative sample 1 was Alldays® Flexible, available from Proctor & Gamble, and Comparative example 2 was Evax , available from Proctor & Gamble.

The training of the panelists was according to the ISO 8586/1 norm. The final assessment was conducted according to the ISO 4121 norm.

Intensity scores were rated on a 10 cm line scales and can be interpreted as follow:

| from [0 to 2] | very low intensity |
|---|---|
| from [2 to 4] | low intensity |
| from [4 to 5] | medium low intensity |
| from [5 to 6] | medium high intensity |
| from [6 to 8] | high intensity |
| from [8 to 10] | very high intensity |

For blood inoculation, the pantiliners were inoculated with natural bacteria species of vaginal flora and pathogen opportunist bacteria in suspension in lamb's blood: 10$^6$ - 10$^7$ cells/ml for *Lactobacillus casei var rhamnosus (Doderlein bacillus)* and 10$^3$ - 10$^4$ cells/ml for *Staphylococcus aureus, Pseudomonas aeruginosa, Proteus mirabilis, Escherichia coli* and *Staphylococcus epidermidis*.

For synthetic urine inoculation, the pantiliners were inoculated with natural bacteria of pubic flora and pathogen opportunist bacteria in suspension in synthetic urine. Synthetic urine used herein was made with 10 g/l NaCl (from CARLO ERBA), 2 g/l (NH4)H$_2$PO$_4$ (from Merck), and 25 g/l urea (from Q-biogène). 10$^6$ - 10$^7$ cells/ml for bacteria *Escherichia coli* and 10$^3$ - 10$^4$ cells/ml for *Staphylococcus aureus*,

*Pseudomonas aeruginosa, Proteus mirabilis* and *Staphylococcus epidermidis*.

Each sample was inoculated on the surface with 5 ml of fluid and conditioned in a sealed plastic bag in order to avoid any evaporation of the inoculums. Incubation temperature was 35°C for both, blood and synthetic urine inoculums.

Descriptive statistics for blood inoculation:

Table 2

| SAMPLE | | ATTRIBUTE | |
|---|---|---|---|
| | | OVERALL SMELL INTENSITY | DAMAGED BLOOD SMELL |
| PANTILINER CONTAINING INSERT | TIME OF INCUBATION | Mean | Mean |
| A CONTROL | 1 HR | 3.2 | 1.7 |
| A CONTROL | 8 HR | 2.8 | 6.8 |
| Option 1 2.5gsm $Na_3$ EDTA (spray) | 1 HR. | 2.8 | 1.5 |
| Option 1 2.5gsm $Na_3$ EDTA (spray) | 8H | 2.8 | 1.9 |
| Option 2a 2.5gsm $Na_3$EDTA (spray) + 12gsm $NaHCO_3$ | 1H | 3.6 | 1.9 |
| Option 2a 2.5gsm $Na_3$EDTA (spray) + 12gsm $NaHCO_3$ | 8H | 3.2 | 1.8 |
| Comparative sample 1 | 1H | 5.5 | 4.1 |
| Comparative sample 1 | 8H | 7.8 | 7.7 |
| Comparative sample 2 | 1H | 5.5 | 4.2 |
| Comparative sample 2 | 8H | 7.4 | 6.6 |

Descriptive statistics for synthetic urine inoculation:

Table 3

| SAMPLE | | ATTRIBUTE | |
|---|---|---|---|
| | | OVERALL SMELL INTENSITY | AMMONIA SMELL |
| PANTILINER CONTAINING INSERT | TIME OF INCUBATION | Mean | Mean |
| A CONTROL | 1H | 3.4 | 1.0 |
| A CONTROL | 8H | 6.9 | 2.5 |
| Option 1 2.5gsm $Na_3$ EDTA (spray) | 1H | 2.9 | 1.3 |
| Option 1 2.5gsm $Na_3$ EDTA (spray) | 8H | 3.7 | 1.3 |
| Option 2a 2.5gsm $Na_3$EDTA (spray) + 12gsm $NaHCO_3$ | 1H | 4.7 | 1.1 |
| Option 2a 2.5gsm $Na_3$EDTA (spray) + 12gsm $NaHCO_3$ | 8H | 3.9 | 0.7 |

Table 3   (continued)

| SAMPLE | | ATTRIBUTE | |
|---|---|---|---|
| | | OVERALL SMELL INTENSITY | AMMONIA SMELL |
| PANTILINER CONTAINING INSERT | TIME OF INCUBATION | Mean | Mean |
| Comparative sample 1 | 1H | 2.9 | 0.6 |
| Comparative sample 1 | 8H | 8.0 | 3.3 |
| Comparative sample 2 | 1H | 3.0 | 1.0 |
| Comparative sample 2 | 8H | 7.5 | 3.1 |

In all cases, the odor rating after 8 hours for the pantiliner products with insert Option 1 and Option 2a was significantly lower compared to the pantiliner products with insert A CONTROL to Comparative sample 1 and Comparative sample 2.

**Claims**

1.   An absorbent article comprising an odor control coating comprising an effective amount of an odor control agent.

2.   An absorbent article of claim 1, wherein the odor control agent is selected from EDTA, a sodium salt thereof, a potassium salt thereof, and an ammonium salt thereof.

3.   An absorbent article of claim 2, wherein the sodium salt is trisodium-EDTA, the potassium salt tripotasium EDTA, and the ammonium salt is triammonium EDTA.

4.   An absorbent article of claim 2, wherein trisodium-EDTA is the odor control agent.

5.   An absorbent article of claim 1, wherein the odor control coating is applied to the absorbent article in an amount sufficient to provide at least about 2.5 gsm of the odor control agent.

6.   An absorbent article of claim 4, wherein the odor control coating is applied to the abosorbent article in an amount of at least about 5 gsm of the odor control agent.

7.   An absorbent article of claim 1, wherein the odor control coating is applied to the cover or the absorbent core.

8.   An absorbent article of claim 7 wherein the odor control coating is applied to the absorbent core.

9.   An absorbent article of claim 2 wherein the odor control coating is applied to the cover or the absorbent core.

10.  An absorbent article of claim 9 wherein the odor control coating is applied to the absorbent core.

11.  An absorbent article of claim 4 wherein the odor control coating is applied to the cover or the absorbent core.

12.  An absorbent article of claim 11 wherein the odor control coating is applied to the absorbent core.

13.  An absorbent article of claim 5 wherein the odor control coating is applied to the cover or the absorbent core.

14.  An absorbent article of claim 13 wherein the odor control coating is applied to the absorbent core.

15.  An absorbent article of claim 6 wherein the odor control coating is applied to the cover or the absorbent core.

16.  An absorbent article of claim 15 wherein the odor control coating is applied to the absorbent core.

17. An absorbent article of claim 1, wherein the absorbent article is selected from the group consisting of a sanitary napkin, a diaper, an incontinence device, a breast pad, an underarm shield, a shoe liner, a surgical dressing, and an adhesive bandage.

18. An absorbent article of claim 17, wherein the absorbent article is a sanitary napkin.

19. An absorbent article of claim 3, wherein the absorbent article is selected from the group consisting of a sanitary napkin, a diaper, an incontinence device, a breast pad, an underarm shield, a shoe liner, a surgical dressing, and an adhesive bandage.

20. An absorbent article of claim 19, wherein the absorbent article is a sanitary napkin.

# FIG. 1A

# FIG. 1B

# FIG. 2A

# FIG. 2B

# FIG.3